# EUROPEAN PATENT APPLICATION

(11) **EP 2 202 319 A1**
(43) Date of publication of application: **30.06.2010**
(21) Application number: 08842746.3
(22) Date of filing: 17.10.2008
(51) Int. Cl.: C12Q 1/68, C12N 15/09, G01N 33/53

(54) **NUCLEIC ACID PROBE, AND METHOD FOR PRODUCTION OF PROBE POLYMER**

(30) Priority: 24.10.2007 JP 2007276681
(71) Applicant: Eisai R&D Management Co., Ltd., Tokyo 112-8088 (JP)
(72) Inventor: USUI, Mitsugu, Kawasaki-shi Kanagawa 210-0855 (JP); HAKII, Chikako, Kawasaki-shi Kanagawa 210-0855 (JP)
(74) Representative: Grünecker, Kinkeldey, Stockmair & Schwanhäusser Anwaltssozietät
(86) International application number: PCT/JP2008/068849
(87) International publication number: WO 2009/054320

(57) **Abstract**

Provided are a method of forming a probe-polymer capable of readily and efficiently forming a polymer of a nucleic acid probe, a probe-polymer formed by the method, and a novel nucleic acid probe used in the method, and a detection method for a target analyte capable of sensitively and readily detecting the target analyte. The nucleic acid probe is formed by including three or more nucleic acid regions, in which each of the nucleic acid regions includes a first region and a second region complementary to the first region, the both regions being adjacent to each other. The polymer of the nucleic acid probe is formed by reacting the nucleic acid probe.

## Description

### Technical Field

The present invention relates to a novel nucleic acid probe, a method of forming a probe-polymer which can form a self-assembly substance (polymer) of the nucleic acid probe to improve the detection sensitivity of a target analyte, a probe-polymer formed by the method, and a detection method for a target analyte by the method.

### Background Art

The inventors of the present invention have proposed a method of forming an assembly substance (polymer) of nucleic acid probes by the self-assembly reaction of probes (probe alternation link self-assembly reaction) using a plurality of kinds of nucleic acid probes having base sequences complementary to other nucleic acid probes as a novel isothermal nucleic acid amplification method using no enzyme (hereinafter, the method of forming a polymer by the self-assembly reaction of probes is referred to as a PALSAR method) (Patent Documents 1 to 4). Also, the inventors have proposed a method of improving the detection sensitivity of a target gene by using the PALSAR method (Patent Document 5).

Although the above-mentioned method can detect a target gene at high sensitivity, it is necessary to use a plurality of kinds of nucleic acid probes for formation of a signal probe-polymer.
Patent Document 1: JP 3267576 B
Patent Document 2: JP 3310662 B
Patent Document 3: WO 02/31192 A
Patent Document 4: JP 2002-355081 A
Patent Document 5: WO 03/029441 A

### Disclosure of the Invention

### Problems to be solved by the Invention

An object of the present invention is to provide a method of forming a probe-polymer capable of readily and efficiently forming a polymer of a nucleic acid probe, a probe-polymer formed by the method, and a novel nucleic acid probe used in the method, and a detection method for a target analyte capable of sensitively and readily detecting the target analyte.

### Means for solving the Problems

The inventors of the present invention have intensively studied to solve the above-mentioned problems. As a result, the inventors have found that a polymer of a nucleic acid probe can be formed by using only one kind of nucleic acid probe by constructing the nucleic acid probe so as to have one region including a base sequence where bases opposite to each other are arranged in a symmetric fashion starting from the center position of the region so that the bases are complementary to each other in a mirror-image relation (symmetry).

That is, the nucleic acid probe of the present invention is a nucleic acid probe comprising three or more nucleic acid regions, wherein each of the nucleic acid regions in the nucleic acid probe comprises a first region and a second region complementary to the first region, the first region and the second region being adjacent to each other.

The method of forming a probe-polymer of the present invention comprises reacting the nucleic acid probe of the present invention to form a polymer of the nucleic acid probe.

The probe-polymer of the present invention is formed by the method of forming a probe-polymer of the present invention.

The detection method for a target analyte of the present invention comprises: forming a probe-polymer by the method of forming a probe-polymer of the present invention, and detecting the probe-polymer to detect a target analyte.

In the detection method for a target analyte of the present invention it is preferred to form a complex comprising the target analyte, an assist probe, and the probe-polymer by using the assist probe that can specifically bind to the target analyte and comprises the same base sequence as a part or the whole of the base sequence of the nucleic acid probe; and to analyze the complex to detect the target analyte. Note that, in the present description, the assist probe refers to a probe comprising a region that can specifically bind to a target analyte to be detected and comprising the same base sequence as a part or the whole of the base sequence of the above-mentioned nucleic acid probe to be used for formation of a polymer, and serves for linking the target analyte and the probe-polymer.

As the above-mentioned target analyte, there is exemplified at least one kind selected from the group consisting of a nucleic acid, an antigen, an antibody, a receptor, a hapten, an enzyme, a protein, a peptide, a polymer, and a carbohydrate.

When the above-mentioned target analyte is a nucleic acid, it is possible to bind the target nucleic acid to the probe-polymer by constructing the above-mentioned nucleic acid probe so as to comprise a sequence complementary to a part of the sequence of the above-mentioned target nucleic acid (target gene).

### Effects of the Invention

The present invention makes it possible to form a polymer of a nucleic acid probe by using only one kind of the nucleic acid probe. Also, the present invention makes it possible to readily detect a target analyte and to significantly improve the detection sensitivity of the target analyte.

### Brief Description of the Drawings

FIG. 1 is a schematic explanatory diagram illustrating one example of a nucleic acid probe of the present invention.
FIG. 2 is a schematic explanatory diagram illustrating a binding pattern of the nucleic acid probe illustrated in FIG. 1.
FIG. 3 is a schematic explanatory diagram illustrating a probe-polymer formed by the nucleic acid probe illustrated in FIG. 1.
FIG. 4 is a photograph showing the results of Example 1.
FIG. 5 is a photograph showing the results of Example 2.
FIG. 6 is a photograph showing the results of Example 3.

### Description of Symbols

10: a nucleic acid probe of the present invention, 10a, 10b, and 10c: nucleic acid regions, 20: a hydrogen bond, 30: a probe-polymer.

### Best Mode for carrying out the Invention

Embodiments of the present invention are hereinafter described based on the attached drawings. Because the illustrated examples are just for exemplification, various modifications are of course feasible as long as the modifications do not depart from the technical idea of the present invention.

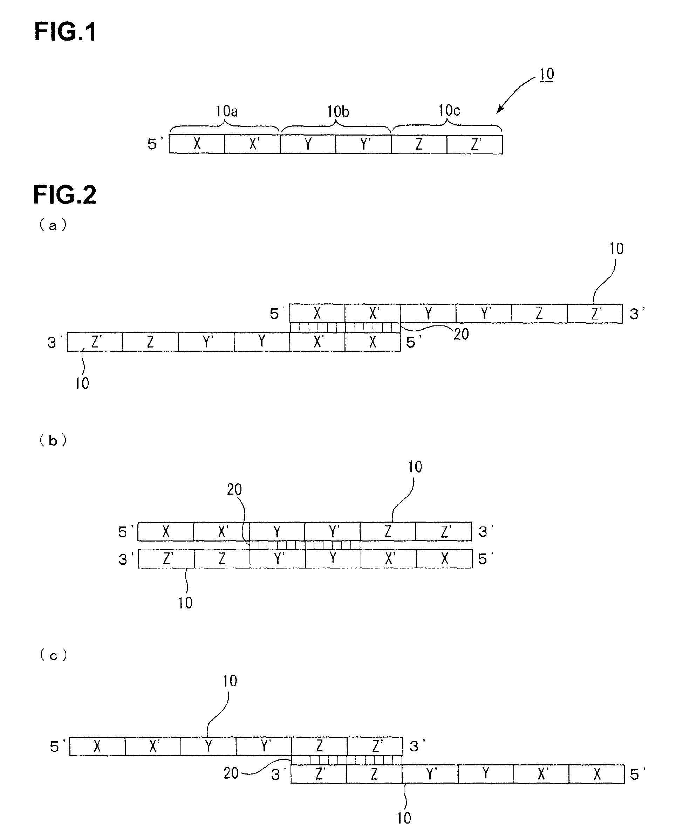

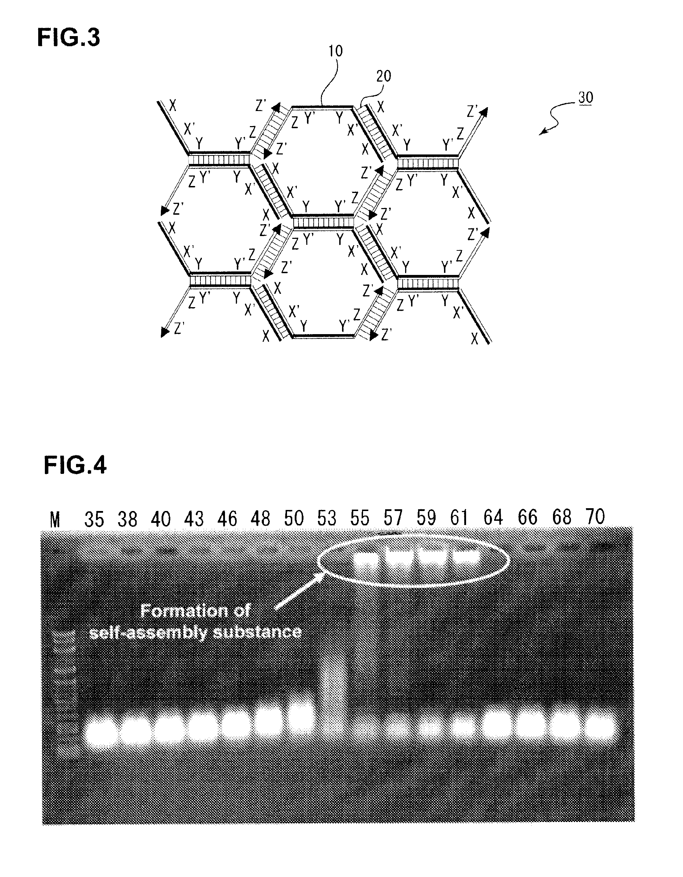

FIG. 1 is a schematic explanatory diagram illustrating one example of the nucleic acid probe of the present invention. FIG. 2 is a schematic explanatory diagram illustrating a binding pattern of the nucleic acid probe illustrated in FIG. 1. FIG. 3 is a schematic explanatory diagram illustrating a self-assembly substance (probe-polymer) formed by using the nucleic acid probe illustrated in FIG. 1.

The nucleic acid probe of the present invention is a nucleic acid probe including three or more nucleic acid regions and is **characterized in that** each of the nucleic acid regions in the nucleic acid probe includes a first region and a second region complementary to the first region, the both regions being adjacent to each other, and a polymer of the nucleic acid probe can be formed by a reaction of the nucleic acid probe. In the present description, the nucleic acid region in the nucleic acid probe means a region including the above-mentioned first and second regions. Meanwhile, each of the first and second regions in each nucleic acid region refers to a complementary region. The number of the nucleic acid regions in the nucleic acid probe of the present invention is 3 or more, preferably 3 or more and 10 or less, more preferably 3 or more and 5 or less.

FIG. 1 is a schematic explanatory diagram illustrating one example of the nucleic acid probe of the present invention and illustrates an example of a nucleic acid probe including three nucleic acid regions. As illustrated in FIG. 1, the nucleic acid probe 10 of the present invention has a nucleic acid region 10a, a nucleic acid region 10b, and a nucleic acid region 10c, which are located in the stated order from the 5'-terminal, and each of the nucleic acid regions 10a, 10b, and 10c includes two regions which are complementary and adjacent to each other. That is, the above-mentioned nucleic acid region 10a includes a complementary region X and a complementary region X' having a base sequence complementary to that of the complementary region X, both regions being adjacent to each other, the above-mentioned nucleic acid region 10b includes a complementary region Y and a complementary region Y' having a base sequence complementary to that of the complementary region Y, both regions being adjacent to each other, and the above-mentioned nucleic acid region 10c includes a complementary region Z and a complementary region Z' having a base sequence complementary to that of the complementary region Z, both regions being adjacent to each other.

In the nucleic acid probe 10 of the present invention, the complementary regions X and X', the complementary regions Y and Y', and the complementary regions Z and Z' are complementary nucleic acid regions capable of hybridizing to each other, and as illustrated in FIG. 2, the complementary region X binds to the complementary region X' [FIG. 2 (a)], the complementary region Y binds to the complementary region Y' [FIG. 2 (b)], and the complementary region Z binds to the complementary region Z' [FIG. 2 (c)]. In FIG. 2, the numeral 20 denotes a hydrogen bond. Note that, although FIG. 1 illustrates the nucleic acid probe having three nucleic acid regions (10a, 10b, and 10c) as a preferred example, the number of the nucleic acid region is not particularly limited as long as the number is three or more.

When a hybridization reaction of the nucleic acid probe 10 of the present invention is performed, as illustrated in FIG. 3, the nucleic acid probe 10 is self-assembled, and as a result, a probe-polymer 30 that is a self-assembly substance of the nucleic acid probe 10 can be formed.

The complementary regions in the nucleic acid probe of the present invention each have a length of preferably 2 bases or more, more preferably from 3 bases to 50 bases, still more preferably from 4 bases to 15 bases. Meanwhile, the complementary regions in the nucleic acid probe desirably each have the same length.

The base sequence of the nucleic acid probe of the present invention is not particularly limited as long as the base sequences of the first and second regions in each nucleic acid region are complementary to each other, and the bases at the both terminals of the complementary regions are preferably guanine or cytosine. If the bases at the both terminals of the complementary regions are guanine or cytosine, it is possible to cut the reaction time, to form a stable probe-polymer at a lower reaction temperature, and to improve the working efficiency and detection sensitivity.

The nucleic acid probe of the present invention is generally constituted of DNA or RNA, and may be constituted of nucleic acid analogs. Examples of the nucleic acid analogs include Peptide Nucleic Acid (PNA, see WO 92/20702 A and the like) and Locked Nucleic Acid (LNA, see Koshkin AA et al. Tetrahedron 1998. 54, 3607-3630., Koshkin AA et al. J. Am. Chem. Soc. 1998. 120, 13252-13253., Wahlestedt C et al. PNAS. 2000. 97, 5633-5638., and the like). Besides, the composition of nucleic acids in the nucleic acid probe is not limited to constitution of one kind of nucleic acid such as only DNA, and, for example, an oligonucleotide probe (chimeric probe) constituted of DNA and RNA can be used as required. Such an oligonucleotide probe is included in the present invention.

Those probes can be synthesized by a known method. For example, DNA probes can be synthesized by a Phosphoamidide method using a DNA synthesizer 394 type manufactured by Applied Biosystem Inc. In addition, a phosphate triester method, an H-phosphonate method, a thiophosphonate method, and the like can be exemplified as other methods, and the probes synthesized by any method can be used.

The method of forming a probe-polymer of the present invention is a method in which the probe-polymer is formed by reacting the nucleic acid probes of the present invention with each other. As illustrated in FIG. 3, a hybridization reaction of the plurality of nucleic acid probes 10 of the present invention leads to efficient formation of a probe-polymer 30, which is a self-assembly substance of the nucleic acid probes 10, depending on the concentration of probes.

The number of the nucleic acid probes used is not particularly limited, and the number of the nucleic acid probes used is in the range of 10² to 10¹⁵. The composition and concentration of a reaction buffer are not particularly limited, and a common buffer usually used for amplification of nucleic acids can be suitably used. A usual range of the pH of a reaction buffer is suitable, and a reaction buffer having a pH in the range of 7.0 to 9.0 can be preferably used. The temperature condition of the hybridization reaction is also not particularly limited, and a usual temperature condition is appropriate. Moreover, it is preferred that a reaction temperature region be partially formed in a reaction solution, and a self-assembly reaction be performed in the reaction temperature region (WO 2005/106031 A). Reaction temperature applied in the reaction temperature region partially formed is preferably 40 to 80°C, more preferably 55 to 65°C.

When a target analyte is detected by the method of forming a probe-polymer of the present invention, the target analyte can be detected readily and sensitively. The detection method for a target analyte is not particularly limited and may be a known method.

The detection method for a target analyte of the present invention includes forming a polymer by using the above-mentioned method of forming a probe-polymer of the present invention, and detecting the polymer to detect the existence of the target analyte in a sample. Specific examples of the above-mentioned detection method for a target analyte include a detection method for a target analyte involving forming a complex of the target analyte and a probe-polymer and detecting the probe-polymer, and a detection method for a target analyte involving forming a polymer by a method of forming a polymer only when the target analyte is present, and detecting the polymer (for example, a method of forming a polymer involving using nucleic acid probe fragments and ligating the fragments through a ligation reaction only when the target analyte is present).

As a sample for measuring a target analyte in the present invention, any sample having a possibility of containing the target analyte can be applied. Examples of the sample include samples derived from living organisms such as blood, serum, urine, feces, cerebrospinal fluid, tissue fluid, sputum, and cell culture, and samples possibly containing or being infected by viruses, bacteria, molds, and the like.
Examples of the target analyte include a nucleic acid, an antigen, an antibody, a receptor, a hapten, an enzyme, a protein, a peptide, a polymer, a carbohydrate, and a combination thereof. The target analyte may be one suitably prepared from a sample or one isolated from a sample. Besides, nucleic acids such as DNA and RNA can also be used, the nucleic acids being obtained by amplifying a target nucleic acid in a sample by a known method. As the target nucleic acid (target gene), single-strand DNA and/or RNA and double-strand DNA and/or RNA can be used. In addition, SNPs (single nucleotide polymorphism) can be used as the target nucleic acid.

In the detection method of the present invention, it is preferred to use an assist probe which has one or two or more regions having the same base sequence as a part or the whole of the base sequence of the nucleic acid probe of the present invention (referred to as probe-binding regions) and has a region that can specifically bind to the target analyte (referred to as target region T). The target analyte can be more readily detected by forming a complex including the target analyte, the assist probe and the polymer by using the assist probe, and analyzing the complex to detect the target analyte. The target region is preferably located on the terminal of the assist probe.

The above-mentioned target region may be suitably selected depending on the target analyte. For example, when the target analyte is a nucleic acid, it is preferred that the target region be constructed so as to have a base sequence complementary to the base sequence of the target nucleic acid, and an assist probe having a sequence complementary to that of one region of the target nucleic acid be used, and their binding be performed by hybridization.
When the target analyte is a protein such as an antigen, the target analyte preferably binds directly or indirectly to a substance capable of specifically binding to the target analyte such as an antibody. Note that the bond of the assist probe and target analyte may be a direct bond of them or an indirect bond via another substance. Specifically, there is preferably used an assist probe obtained by binding an antibody by chemical bonding, such as an assist probe obtained by conjugating an antibody by binding an amino group to a carboxyl group or the like. There may be also used an assist probe obtained by binding a biotinylated antibody with streptavidin.

The probe-binding region of the above-mentioned assist probe is preferably constructed so as to include one or more regions having the same base sequence as that of the complementary region of the nucleic acid probe of the present invention, more preferably constructed so as to include the two or more same kinds of complementary regions as those of the nucleic acid probe of the present invention, to thereby bind the assist probe to the nucleic acid probe of the present invention via two or more consecutive complementary regions. In addition, an assist probe including a plurality of the same probe-binding regions may be used to bind one assist probe to two or more nucleic acid probes.

When the target analyte is a nucleic acid, the target nucleic acid can be detected by constructing the nucleic acid probe of the present invention so as to have a sequence complementary to that of a part of the above-mentioned target nucleic acid, forming a complex of the target nucleic acid and a probe-polymer by using the nucleic acid probe, and detecting the target nucleic acid by detecting the probe-polymer.

Further, in the detection method of the present invention, a step of capturing a target analyte with a reaction material for detecting a target analyte is preferably included. The present invention is applicable to various reaction materials for detecting a target analyte, and is suitably used for a DNA chip, a DNA microarray (see Marshall, A., Hodgson, J. DNA chips: an array of possibilities. Nat Biotechnol. 16, 27-31, 1998 and the like), a microplate, a magnetic particles, and the like.

A method of capturing a target analyte with a reaction material is not particularly limited. A preferred method is a method in which a reaction material obtained by bonding a capturing material capable of specifically bonding to a target analyte is used, and the reaction material and the target analyte are bonded by bonding of the capturing material and the target analyte.
The capturing material may be suitably selected depending on the target analyte and is not particularly limited. When the target analyte is a nucleic acid, an oligonucleotide (capture probe) having the base sequence complementary to one region (excluding the region complementary to that of an assist probe) of the target nucleic acid is preferably used as the capturing material. When the target analyte is an antigen or an antibody, an antibody or an antigen that bonds to the target analyte specifically is preferably used as the capturing material. In addition, when the target analyte is a carbohydrate, lectin that specifically bonds to the target analyte is preferably used as the capturing material.

In the present invention, a detection method for a probe-polymer is not particularly limited. A preferred method is a method in which the nucleic acid probe is preliminarily labeled with a labeling substance and the detection is performed by using the labeling substance. The labeling substance is preferably acridinium ester, a radioisotope, biotin, digoxigenin, a fluorescent substance, a luminescent substance, or pigment. Acridinium ester is particularly preferred in consideration of its operability, quantitative capability, and sensitivity. To be specific, the nucleic acid probe of the present invention is preliminarily labeled with a fluorescent substance, and the existence of the probe-polymer can be detected based on a photochemical change of the fluorescent substance. In addition, the nucleic acid probe of the present invention is preliminarily labeled with a chromogenic enzyme or a luminescent enzyme, and the existence of the probe-polymer can be detected based on a photochemical change. Moreover, the nucleic acid probe of the present invention is preliminarily labeled with a radioisotope, and the existence of the probe-polymer can be detected with the radioisotope.

Further, the existence of the formed probe-polymer can be detected by hybridizing a labeled probe to the probe-polymer. As the labeled probe, there may be used a substance labeled with a chromogenic enzyme, a luminescent enzyme, a radioisotope, or the like. Besides, a fluorescent substance having a characteristic of binding to a nucleic acid is added to the formed probe-polymer, and the existence of the probe-polymer can be detected based on a photochemical change of the fluorescent substance. As the fluorescent substance, a fluorescent substance having a characteristic of being insertable into a double-stranded base pair is preferred.

In addition, the probe-polymer of the present invention expresses a hypochromic effect called "hypochromism" to an extremely large degree, the hypochromism being a phenomenon that the intensity of an absorption band of an ultraviolet portion at 260 nm decreases. Therefore, the state of the polymer can be confirmed by measuring the absorption of the ultraviolet portion at a wavelength of 260 nm.

### Examples

Hereinafter, the present invention is described more specifically by way of examples, but needless to say, these examples are illustrative only and should not be construed restrictively.

### (Example 1)

Electrophoresis was performed to confirm whether or not a probe-polymer was formed by the nucleic acid probe of the present invention.

A nucleic acid probe-1 (SEQ ID NO: 1) having the following base sequence was dissolved in a reaction solution [4XSSC] so as to have a concentration of 2000 pmol/mL, to thereby prepare a probe solution.
Base sequence of nucleic acid probe-1 (5'-X₁ (base number 7) X₁' (base number 7)-Y₁ (base number 7) Y₁' (base number 7)-Z₁ (base number 7) Z₁' (base number 7)-3')
5'-CCTGTCT AGACAGG CTTCGGA TCCGAAG GGTAGCA TGCTACC-3' (SEQ ID NO: 1)

The probe solution prepared above was heated at 94°C for 1 minute and immediately cooled on ice. Subsequently, a tube was set at the state where the temperature of the tube preliminarily reached at each reaction temperature [35, 38, 40, 43, 46, 48, 50, 53, 55, 57, 59, 61, 64, 66, 68, or 70°C] to undergo a reaction for 1 hour. After the reaction, the samples were electrophoresed on a 0.25% agarose gel. The results are shown in FIG. 4. In FIGS. 4 to 6, M denotes a molecular size marker, and each numeral represents a reaction temperature condition.
As shown in FIG. 4, probe-polymers were formed at reaction temperatures of 55°C to 61°C.

### (Example 2)

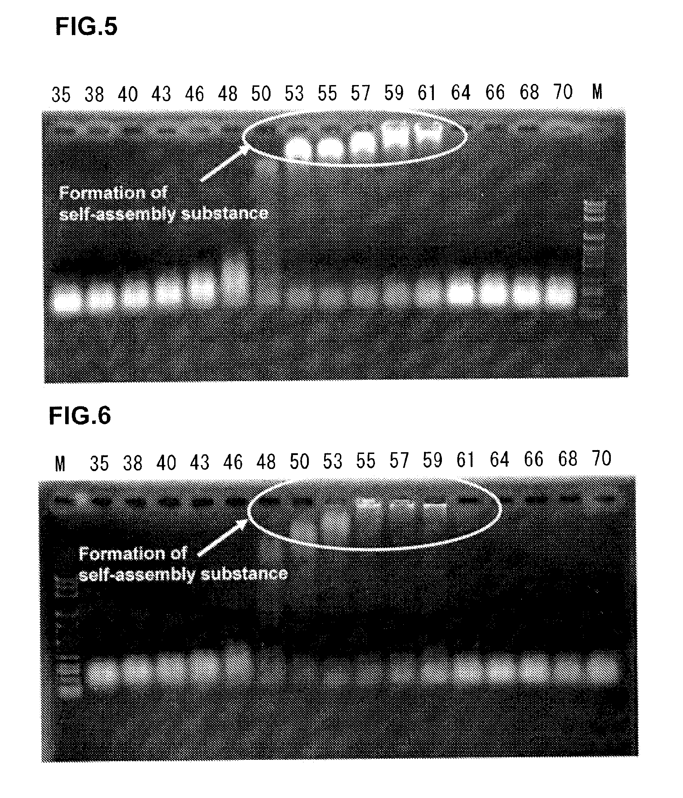

The same experiment as in Example 1 was performed except that a nucleic acid probe-2 having the following base sequence (SEQ ID NO: 2) was used instead of the nucleic acid probe-1. The results are shown in FIG. 5.
Base sequence of nucleic acid probe-2 (5'-X₂ (base number 6) X₂' (base number 6)-Y₂ (base number 6) Y₂' (base number 6)-Z₂ (base number 6) Z₂' (base number 6)-3')
5'-CCTGTC GACAGG CTCGGA TCCGAG GGAGCA TGCTCC-3' (SEQ ID NO: 2)
As shown in FIG. 5, probe-polymers were formed at reaction temperatures of 53°C to 61°C.

### (Example 3)

The same experiment as in Example 1 was performed except that a nucleic acid probe-3 having the following base sequence (SEQ ID NO: 3) was used instead of the nucleic acid probe-1. The results are shown in FIG. 6.
Base sequence of nucleic acid probe-3 (5'-X₃ (base number 5) X₃' (base number 5)-Y₃ (base number 5) Y₃' (base number 5)-Z₃ (base number 5) Z₃' (base number 5)-3')
5'-CCTGC GCAGG CTCGG CCGAG GGACG CGTCC-3' (SEQ ID NO: 3)

As shown in FIG. 6, probe-polymers were formed at reaction temperatures of 50°C to 59°C.

### (Example 4)

A target gene was detected by forming a probe-polymer using the nucleic acid probe of the present invention.

A synthetic DNA (target oligo DNA) (SEQ ID NO: 4) having the same base sequence as that of rRNA of *Staphylococcus aureus* was used as a target analyte.

Base sequence of target oligo DNA

5'-TTCGGGAAACCGGAGCTAATA CCGGATAATATTTTGAACCGC ATGG
TTCAAAAGTGAAAGACG GTCTTGCTGTCACTTATAGAT GGATCCGCGC
TGCATTAGCTA-3' (SEQ ID NO: 4)

A nucleic acid probe (SEQ ID NO: 5) having a sequence complementary to that of the above-mentioned target oligo DNA (SEQ ID NO: 4) was used as a capture probe.

Base sequence of capture probe
5'-CGTCTTTCACTTTTGAACCAT GCGGTTCAAAATATTATCCGG-3'-Amino link (SEQ ID NO: 5)

The above-mentioned capture probe was immobilized on each well of a 96-well microplate of strip well-type and used for the experiment.

A nucleic acid probe obtained by labeling the 5'-terminal of the nucleic acid probe-1 (SEQ ID NO: 1) used in Example 1 with acridinium ester (AE) was used as a nucleic acid probe to be used for formation of a probe-polymer. The nucleic acid probe-1 labeled with AE was dissolved in a solution [100 mM-lithium succinate, 600 mM-lithium chloride, 2 mM-EDTA, 5%-LDS, a stabilizer] so as to have a concentration of 50 pmol/mL to prepare a second hybridization solution.

A nucleic acid probe (assist probe-1) (SEQ ID NO: 6) having the same base sequence as a part of the base sequence of the above-mentioned nucleic acid probe-1 and a region complementary to the above-mentioned target oligo DNA was used as the assist probe.
Base sequence of assist probe-1 (5'-Y₁'-X₁-polyT-Y₁'-X₁-T'-3')

5'-TCCGAAG CCTGTCT TTTTT TCCGAAG CCTGTCT ATCTATAAGTGA
CAGCAAGAC-3' (SEQ ID NO: 6)

The above-mentioned assist probe was dissolved in a solution [100 mM-lithium succinate, 600 mM-sodium chloride, 2 mM-EDTA, 1%-LDS] so as to have a concentration of 25 pmol/mL to prepare a first hybridization solution.

To each well of the prepared 96-well microplate of strip well type, 50 µL of 0 or 10 fmol/mL target oligo DNA (SEQ ID NO: 4) and 50 µL of the first hybridization solution were fed, and the microplate was tightly sealed with a plate-sealer, and then was subjected to a reaction for 45 minutes under a condition of at 20°C in the upper part of the microplate and at 55°C in the lower part of the microplate. After the reaction, the microplate was washed with a washing solution [50 mM-Tris, 0.3 M-NaCl, 0.01%-Triton X-100, pH 7.0].

After the washing, the washing solution was fully removed from the 96-well microplate. To each well of the microplate, 100 µL of the second hybridization solution were fed, and the microplate was tightly sealed with a plate-sealer. The microplate was subjected to a reaction for 30 minutes under a condition of at 20°C in the upper part of the microplate and at 55°C in the lower part of the microplate. After the reaction, the microplate washed with the washing solution.

After the microplate wells were washed, 50 µL of each a luminescent reagent-I and II (manufactured by Gen-Probe Incorporated) were added thereto, and relative light units (RLU) were measured with a luminometer (Centro LB960, manufactured by BERTHOLD TECHNOLOGIES GmbH & Co. KG). The measurement results of the relative light units (RLU) are shown in Table 1.

### (Example 5)

The same experiment as in Example 4 was performed except that the conditions were changed as follows. The results are shown in Table 1.
A nucleic acid probe obtained by labeling the 5'-terminal of the nucleic acid probe-3 (SEQ ID NO: 3) used in Example 3 with acridinium ester (AE) was used as a nucleic acid probe to be used for formation of a probe-polymer.
A nucleic acid probe (assist probe-2) (SEQ ID NO: 7) having the same base sequence as a part of the base sequence of the above-mentioned nucleic acid probe-3 and a region complementary to the above-mentioned target oligo DNA was used as the assist probe.
Base sequence of assist probe-2 (5'-Y₃'-X₃-X₃'-Y₃'-X₃-X₃'-T'-3')

5'-CCGAG CCTGC GCAGG CCGAG CCTGC GCAGG ATCTATAAGTGAC
AGCAAGAC-3' (SEQ ID NO: 7)

### (Comparative Example 1)

A target gene was detected by a method of forming a probe-polymer using two kinds of nucleic acid probes.
The same experiment as in Example 4 was performed except that the conditions were changed as follows. The results are shown in Table 1.
The following two kinds of nucleic acid probes (first and second probes; SEQ ID NOS: 8 and 9) the 5'-terminals of which were labeled with acridinium ester (AE) were used as nucleic acid probes to be used for formation of a probe-polymer. The two kinds of nucleic acid probes labeled with AE were separately dissolved in a solution [100 mM-lithium succinate, 600 mM-lithium chloride, 2 mM-EDTA, 5%-LDS, a stabilizer] so as to have a concentration of 25 pmol/mL to prepare a second hybridization solution.
Base sequence of first probe (5'-X₄-Y₄-Z₄-3')
5'-GATGTCTCGGGATG GCTTCGGAGTTACG CTGGCGGTATCAAC-3' (SEQ ID NO: 8)
Base sequence of second probe (5'-X₄'-Y₄'-Z₄'-3')
5'-CATCCCGAGACATC CGTAACTCCGAAGC GTTGATACCGCCAG-3' (SEQ ID NO: 9)

A nucleic acid probe (assist probe-3) (SEQ ID NO: 10) having the same base sequence as a part of the base sequence of the above-mentioned first probe and a region complementary to the above-mentioned target oligo DNA was used as the assist probe.
Base sequence of assist probe-3 (5'-X₄-Y₄-X₄-T'-3')

5'-GATGTCTCGGGATG GCTTCGGAGTTACG GATGTCTCGGGATG ATC
TATAAGTGACAGCAAGAC-3' (SEQ ID NO: 10)

**[Table 1]**

| Target oligo DNA concentration (fmol/mL) | Example 4 | Example 5 | Comparative Example 1 |
|---|---|---|---|
| 0 | 98 | 38 | 148 |
| 10 | 84,632 | 38,430 | 82,496 |

As shown in Table 1, even in the case where one kind of nucleic acid probe was used, the target was able to be detected as in the case of using the two kinds of nucleic acid probes.

## Claims

1. A nucleic acid probe, comprising three or more nucleic acid regions, wherein each of the nucleic acid regions in the nucleic acid probe comprises a first region and a second region complementary to the first region, the first region and the second region being adjacent to each other.

2. A method of forming a probe-polymer, comprising reacting the nucleic acid probe according to claim 1 to form a polymer of the nucleic acid probe.

3. A probe-polymer, which is formed by the method according to claim 2.

4. A detection method for a target analyte, comprising:
forming a probe-polymer by the method according to claim 2; and
detecting the probe-polymer to detect a target analyte.

5. A detection method according to claim 4, comprising:
forming a complex comprising the target analyte, an assist probe, and the probe-polymer by using the assist probe that can specifically bind to the target analyte and comprises the same base sequence as a part or the whole of the base sequence of the nucleic acid probe; and
analyzing the complex to detect the target analyte.

6. A detection method according to claim 4, wherein the target analyte is a nucleic acid, and the nucleic acid probe comprises a sequence complementary to a part of the sequence of the target nucleic acid.

7. A detection method according to claim 4 or 5, wherein the target analyte is at least one kind selected from the group consisting of a nucleic acid, an antigen, an antibody, a receptor, a hapten, an enzyme, a protein, a peptide, a polymer, and a carbohydrate.


SEQUENCE LISTING

<110>  Eisai R&D Management Co., Ltd.

<120>  Nucleic acid probe and method for forming probe-polymer

<130>  77547-P-PCT

<150>  JP 2007-276681
<151>  2007-10-24

<160>  10

<170>  PatentIn version 3.1

<210>  1
<211>  42
<212>  DNA
<213>  Artificial

<220>
<223>  Description of Artificial Sequence; Synthetic

<400>  1
cctgtctaga caggcttcgg atccgaaggg tagcatgcta cc                    42


<210>  2
<211>  36
<212>  DNA
<213>  Artificial

<220>
<223>  Description of Artificial Sequence; Synthetic

<400>  2
cctgtcgaca ggctcggatc cgagggagca tgctcc                           36


<210>  3
<211>  30
<212>  DNA
<213>  Artificial

<220>
<223>  Description of Artificial Sequence; Synthetic

<400>  3
cctgcgcagg ctcggccgag ggacgcgtcc                                  30


<210>  4
<211>  105
<212>  DNA
<213>  Artificial

<220>
<223>  Description of Artificial Sequence; Synthetic

<400>  4
ttcgggaaac cggagctaat accggataat attttgaacc gcatggttca aaagtgaaag    60

acggtcttgc tgtcacttat agatggatcc gcgctgcatt agcta 105

<210> 5
<211> 42
<212> DNA
<213> Artificial

<220>
<223> Description of Artificial Sequence; Synthetic

<220>
<221> misc_feature
<222> (42)..(42)
<223> Aminolink attached at the 3'end

<400> 5
cgtctttcac ttttgaacca tgcggttcaa aatattatcc gg 42

<210> 6
<211> 54
<212> DNA
<213> Artificial

<220>
<223> Description of Artificial Sequence; Synthetic

<400> 6
tccgaagcct gtcttttttt ccgaagcctg tctatctata agtgacagca agac 54

<210> 7
<211> 51
<212> DNA
<213> Artificial

<220>
<223> Description of Artificial Sequence; Synthetic

<400> 7
ccgagcctgc gcaggccgag cctgcgcagg atctataagt gacagcaaga c 51

<210> 8
<211> 42
<212> DNA
<213> Artificial

<220>
<223> Description of Artificial Sequence; Synthetic

<400> 8
gatgtctcgg gatggcttcg gagttacgct ggcggtatca ac 42

<210> 9
<211> 42
<212> DNA

<213>  Artificial

<220>
<223>  Description of Artificial Sequence; Synthetic

<400>  9
catcccgaga catccgtaac tccgaagcgt tgataccgcc ag                          42


<210>  10
<211>  63
<212>  DNA
<213>  Artificial

<220>
<223>  Description of Artificial Sequence; Synthetic

<400>  10
gatgtctcgg gatggcttcg gagttacgga tgtctcggga tgatctataa gtgacagcaa      60

gac                                                                     63